# EUROPÄISCHE PATENTSCHRIFT

(11) **EP 0 255 033 B1**
(45) Veröffentlichungstag und Bekanntmachung des Hinweises auf die Patenterteilung: **06.04.1994**
(21) Anmeldenummer: 87110615.9
(22) Anmeldetag: 22.07.1987
(51) Int. Cl.: C07H 15/04, C08F 20/58, C11D 1/52

(54) **Isomaltamine sowie deren N-Acylderivate, Verfahren zu deren Herstellung und ihre Verwendung**
Isomaltamine and its N-acylated derivatives, method for their preparation and their use
Isomaltamine et ses dérivés N-acyles, méthode pour leur préparation et leur utilisation

(30) Priorität: 31.07.1986 DE 3625931
(43) Veröffentlichungstag der Anmeldung: 03.02.1988
(73) Patentinhaber: SÜDZUCKER AKTIENGESELLSCHAFT MANNHEIM/OCHSENFURT, D-68165 Mannheim (DE)
(72) Erfinder: Klein, Joachim, Prof. Dr., D-3300 Braunschweig (DE); Behrens, Wolfgang, D-3180 Wolfsburg 1 (DE); Kunz, Markwart, Dr. rer. nat., D-3300 Braunschweig (DE)
(74) Vertreter: Gleiss, Alf-Olav, Dr.jur. Dipl.-Ing.

(56) Entgegenhaltungen:
- EP-A- 0 114 954
- EP-A- 0 166 089
- EP-A- 0 220 676
- US-A- 2 752 334
- CHEMICAL ABSTRACTS, Band 98, Nr. 22, 30. Mai 1983, Seite 10, ZusammenfassungNr. 180070c, Columbus, Ohio, US; & JP-A-57 202 309 (AGENCY OF INDUSTRIAL SCIENCES AND TECHNOLOGY) 11-12-1982

## Beschreibung

Die vorliegende Erfindung betrifft die im Anspruch 1 angeführten neuen Verbindungen entsprechend den Formeln 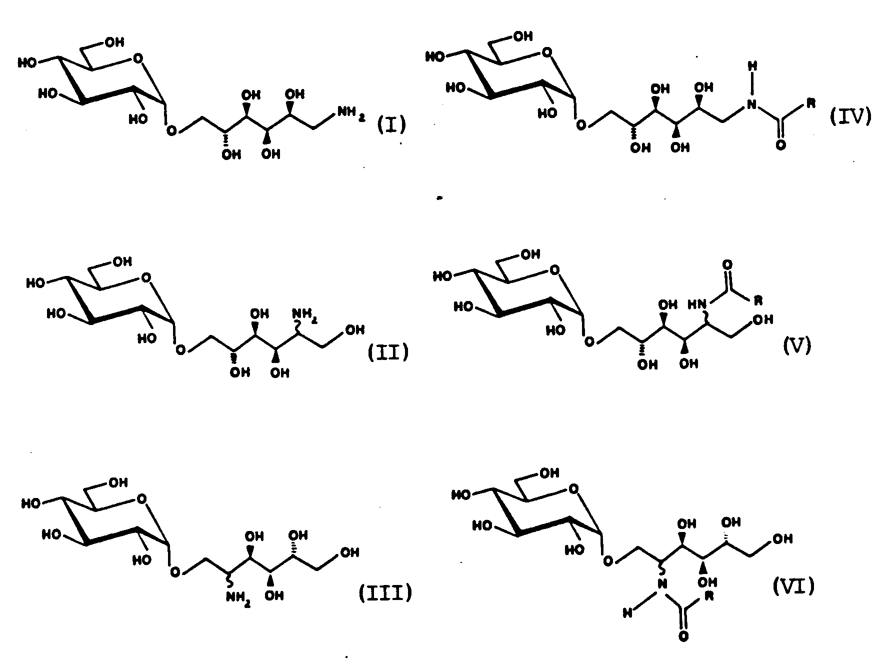
sowie die Herstellung der Isomaltamine der Formeln I bis III aus den Disacchariden Isomaltose, Isomaltulose und α-D-Glucopyranosyl(1→1)-D-fructose, im folgenden auch Trehalulose genannt, ferner die Herstellung und Verwendung der N-Acylderivate entsprechend den angegebenen Formeln IV bis VI, in denen R ein verzweigter oder unverzweigter, gesättigter oder ungesättigter Alkylrest mit mindestens 2 C-Atomen ist.

Aminopolyole, sowie Verfahren zur Herstellung von Aminopolyolen aus reduzierenden Kohlenhydraten sind in der Literatur schon erwähnt (z.B. US-PSen 2,016,962 und 2,830,983 sowie DD-PS 13746).

Die Herstellung der erfindungsgemäßen Isomaltamine entsprechend den Formeln I bis III kann in Anlehnung an in der Literatur erwähnte Verfahren aus Isomaltose, Isomaltulose und Trehalulose mit verschiedenen Aminierungsmitteln, wie Ammoniak, Hydrazin oder Hydrazinderivaten, in verschiedenen Lösungsmitteln, z.B. Wasser, Methanol, oder Mischungen aus Wasser und Methanol, mit verschiedenen Reduktionsmethoden entweder katalytisch, z.B. in Gegenwart von Kupfer-, Platin- oder Palladium-Katalysatoren und vorzugsweise mit Raney-Nickel und Wasserstoff, oder mit komplexen Metallhydriden, wie Natriumborhydrid, erfolgen.

### Die Bezeichnung für die erfindungsgemäßen Isomeren gemäß Formel I, II und III ist:

Formel I =
α-D-1-Desoxy-1-amino-glucopyranosyl-(1ʹ→6)-D-sorbit; als Kurzbezeichnung empfiehlt sich 1-Amino-(1ʹ→6)-GPS oder 1ʹ→6-Isomaltamin-(1).

Formel II =
α-D-2-Desoxy-2-amino-glucopyranosyl-(1ʹ→6)-D-sorbit (mit der Kurzbezeichnung 2-Amino-(1ʹ→6)-GPS) bzw.
α-D-2-Desoxy-2-amino-glucopyranosyl-(1ʹ→6)-D-mannit (mit der Kurzbezeichnung 2-Amino-(1ʹ→6)-GPM) für das Isomere.

Ein Gemisch der beiden Isomeren (gemäß Formel II) wäre in Anlehnung an den generic name (Isomalt) des Hydrierungsproduktes der Isomaltulose als 2-Desoxy-2-amino-(1ʹ→6)-Isomalt oder verkürzt als 1ʹ→6-Isomaltamin-(2) zu bezeichnen.

Formel III=
α-D-2-Desoxy-2-amino-glucopyranosyl-(1ʹ→1)-D-sorbit (mit der Kurzbezeichnung 2-Amino-(1ʹ→1)-GPS) bzw.
α-D-2-Desoxy-2-amino-glucopyranosyl-(1ʹ→1)D-mannit (mit der Kurzbezeichnung 2-Amino-(1ʹ→1)-GPM) für das Isomere.

Ein Gemisch der beiden Isomeren wäre als 2-Desoxy-2-amino-(1ʹ→1)-isomalt, oder verkürzt als 1ʹ→1-Isomaltamin-(2) zu bezeichnen.

Die Konstitution der vorgenannten Amine ist durch NMR-Spektren gesichert.
Die Isomeren unterscheiden sich, ähnlich wie Mannit und Sorbit bzw. Glucopyranosyl-(1ʹ→6)-mannit und -sorbit, wesentlich in ihrer Löslichkeit. Zum Beispiel sind 1- und 2-Amino-(1ʹ→6)-GPS gut in wasserfreiem Methanol löslich, während 2-Amino-(1ʹ→6)-GPM beim Versetzen konzentrierter wäßriger Lösungen mit Methanol ausfällt. Entsprechendes Lösungsverhalten zeigen die Isomeren gemäß Formel III.

Isomaltulose als eines der Edukte für die erfindungsgemäßen Aminopolyole wird durch enzymatische Transglucosidierung großtechnisch in hochreiner kristalliner Form dargestellt (s. H. Schiweck in Ullmann's Encyclopädie der Technischen Chemie, 4. Aufl., Bd. 24, S. 780 ff.). Dagegen enthält beispielsweise handelsübliche Maltose mehr oder weniger große Anteile Glucose und höhere Maltooligomere. Auch Trehalulose wird durch enzymatische Transglucosidierung aus Saccharose dargestellt (H. Schiweck, s. o.).

Bei der Herstellung und Aufarbeitung der erfindungsgemäßen Aminopolyole hat sich überraschenderweise herausgestellt, daß sie sich problemlos ohne Zersetzung mit sauren Ionenaustauschern von neutralen Produkten trennen lassen, während andere Aminopolyole, wie Maltamin, bei gleicher Behandlung partiell zu Glucamin und anderen Produkten zersetzt werden.

Nicht nur chemische, auch mikrobiologische Stabilität ist bei einer Reihe von Anwendungen, insbesondere bei technischen Hilfsstoffen, z. B. in der Tertiärförderung von Erdöl, erwünscht. Bei der Untersuchung zur biologischen Abbaubarkeit zeigten die erfindungsgemäßen Aminopolyole überraschenderweise hohe mikrobiologische Stabilität.

Als besonders schonendes Verfahren zur Herstellung möglichst gering gefärbter Aminopolyole aus reduzierenden Kohlenhydraten hat sich die reduktive Aminierung mit Hydrazin und Raney-Nickel als Katalysator für Monosaccharide bewährt. Dennoch lassen sich, insbesondere bei Oligosacchariden mit α-glycosidischer Bindung, wie Maltose, Vefärbung und Zersetzung nicht ganz vermeiden.

Im Gegensatz dazu sind die erfindungsgemäß dargestellten Aminopolyole entsprechend den Formeln, I,II und III überraschenderweise nicht gefärbt und so stabil, daß sie auch mit stark sauren Kationenaustauschern gereinigt werden können.

In einigen Anwendungen muß Einheitlichkeit der Produkte nicht zwingend erforderlich sein. Mischungen der erfindungsgemäßen Aminopolyole lassen sich problemlos auch aus Zwischen- und Endprodukten der Palatinoseherstellung gewinnen. Je nach Zusammensetzung des Zwischen- bzw. Endproduktes bestehen die Aminopolyolgemische aus unterschiedlichen Anteilen der Verbindungen gemäß Formeln I - III.

N-Derivate von Kohlehydraten finden zunehmendes Interesse in einer Reihe von Anwendungen. So gibt es etwa in der Medizin Beispiele von pharmakologisch wirksamen N-Derivaten von Kohlehydraten (z.B. DE-OS 34 05 841 oder EP 0 096 392 B1). In der Kosmetik finden N-Derivate von Aminopolyolen vorrangig aufgrund ihrer feuchtigkeitsstabilisierenden Eigenschaften oder ihrer Tensidwirkung Anwendung (z.B. DE-OSen 25 33 101, 12 61 861 oder JP 59.212.419).
Die erfindungsgemäßen Aminopolyole bzw. ihre Derivate haben z.T. ebenfalls feuchtigkeitsstabilisierende bzw. oberflächenaktive Eigenschaften.
Besonders eignen sie sich zur Darstellung von hydrophilen bzw. amphiphilen N-Acylderivaten gemäß den Formeln IV, V und VI.
Diese N-Acylate können, wenn der Acylteil funktionelle Gruppen und/oder Doppelbindungen enthält, als Zwischenprodukte oder z.B., wenn der Acylteil eine langkettige Alkylgruppe enthält, als Endprodukte geeignet sein.

Überraschenderweise lassen sich z.B. aus den erfindungsgemäßen Aminopolyolen dargestellte N-Acrylate und N-Methacrylate problemlos kristallisieren, selbst wenn Mischungen der Isomeren, z.B. entsprechend Formel II, eingesetzt werden. Aus Maltamin dargestellte vergleichbare N-Acylate konnten dagegen bisher nicht kristallisiert werden. Je nach Acylrest eignen sich die Derivate z.B. als Tenside oder zur Herstellung von Polymeren.

N-Acylate gemäß den Formeln IV, V und VI mit Alkylresten zwischen 5 und 21 Kohlenstoffatome sind ausgeprägt amphiphil. Ihre HLB-Werte berechnen sich zu ca. 10 bis ca. 15. Amphiphile mit diesen HLB-Werten (hydrophillc lipophilic balance) finden von Netzmitteln über "Öl-in-Wasser-Emulgatoren" bis hin zu waschaktiven Substanzen Anwendung (Das Atlas-HLB-System, Atlas Chemie GmbH; Essen, 1968).

Vergleichbaren Amphiphilen, z.B. Saccharosemonoestern, sind die erfindungsgemäßen Amide aufgrund ihrer höheren Stabilität sowohl hinsichtlich der glykosidischen Bindung als auch der Amidbindung gegenüber der Esterbindung überlegen. Auch lassen sich z.B. reine Saccharosemonoester nur unter großem Aufwand von höher veresterten Produkten trennen, während die erfindungsgemäßen Amide reine Monoderivate sind (zu Saccharoseestern: G. Schuster, Emulgatoren für Lebensmittel, Springer Verlag Berlin Heidelberg N. Y. 1985, S. 142 ff.).

Wasserlösliche bzw. hydrophile Polymere haben vielseitige Anwendungen. Sie werden als Dickungsmittel in der Technik, Pharma- and Nahrungsmitttelindustrie, zur Tertiärförderung von Erdöl, aber auch als Hilfsmittel für Textil- und Papierherstellung benötigt (vgl. z. B. Chemistry and Technology of water-soluble Polymers, edited by C. A. Finch Plenum Press New York 1983, S. 11 ff).

Aus Aminopolyol-N-acrylaten und N-methacrylaten lassen sich bei entsprechender Reinheit der Derivate hochmolekulare Poly-Vinyl-Saccharide darstellen (J. Klein; D. Herzog; Veröffentlichung in Vorbereitung; Macromol. Chem. Rap. Comm. 1986). Wegen der schwierigen Isolierung von reinen nicht gefärbten Aminopolyolen aus reduzierenden Oligosacchariden, wie Maltamin, konnten bislang keine hochmolekularen Aminopolyolacrylamide aus Oligosacchariden erhalten werden.

Erfindungsgemäß dargestellte N-Acylate der Acryl-, Methacryl-, Itacon- und Malein-Säure fallen in besonders reiner Form frei von störenden braunen Begleitstoffen an, so daß sie sich in ausgezeichneter Weise zur Darstellung hochmolekularer Homo- und Copolymerer eignen.

Diese Polymere zeigten bei biologischen Abbauversuchen im Gegensatz zu vielen natürlichen Polysacchariden überraschenderweise hohe mikrobiologische Stabilität.

Durch Homo- oder Copolymerisation aus den erfindungsgemäßen Amiden dargestellte Polymere sind daher anderen mikrobiologisch oder chemisch weniger stabilen hydrophilen Polymeren in einer Reihe von Anwendungen, insbesondere zur Tertiärförderung von Erdöl, überlegen.

Die Beispiele 1 - 5 beschreiben die Herstellung und Aufarbeitung von Verbindungen gemäß den Formeln I - III aus Isomaltose, Isomaltulose bzw. Trehalulose.

### Beispiel 1

Katalytische reduktive Aminierung der Disaccharide mit Hydrazin.

150 g Isomaltose bzw. Isomaltulose oder Trehalulose werden bei Raumtemperatur in ca. 400 ml Wasser gelöst und nach Zugabe von ca. 50 ml 80%igem Hydrazinhydrat 6 - 12 Stunden bei Raumtemperatur gerührt. Die so erhaltene Lösung wird in einem Hochdruckautoklaven in Gegenwart von 20 g feuchtem Raney-Nickel bei ca. 100 -150 bar Wasserstoffdruck und einer Temperatur von ca. 50°C hydriert. Wichtig ist gute Rührung und Temperierung. Nach 6 - 18 Stunden wird das Reaktionsgemisch dem Autoklaven entnommen und der Hydrierkatalysator abfiltiert. Gelöstes Nickel kann mit geeigneten Reagentien, wie Diacetyldioxim, durch Fällung und Filtration entfernt werden. Für technische Darstellung empfiehlt sich eine Entfernung des Nickels mit einem selektiven organischen Metallextraktionsöl in einer Emulsion und nachfolgender Phasentrennung.

In analoger Weise kann die Hydrierung mit anderen Katalysatoren, z. B. auf Kupfer-, Palladium- oder Platinbasis, durchgeführt werden. Die Ausbeuten an Aminosaccharid sind aber deutlich geringer (<20%), eine Aufarbeitung über Ionenaustauscher (s. unten) ist zwingend erforderlich.

Je nach Verwendungszweck der Aminopolyole kann die Aufarbeitung nach 2 Methoden durchgeführt werden:
- Reinigung über Ionenaustauscher
   Die Lösungen der Rohprodukte enthalten im allgemeinen geringe Anteile nicht umgesetzte Edukte sowie hydrierte Polyole. Diese können, falls hohe Produktreinheit gewünscht wird, durch Ionenaustauscherbehandlung entfernt werden. Dazu wird das klare Filtrat der vom Katalysator befreiten Lösung bis zur Trockne eingeengt. Der Rückstand (ca. 140 g) wird nach Lösen in wenig Wasser auf eine Kationenaustauschersäule (z. B. 2,3 l IR 120, Fa. Amberlite, (H⁺- Form) gegeben, die Säule mit ca. 10 l Wasser zuckerfrei gewaschen und mit dem fünffachen Bettvolumen einer 5%igen wäßrigen Ammoniaklösung eluiert. Aus dem Eluat wird das Aminopolyol durch Destillation bis zur Trockne gewonnen (Ausbeute ca. 85%).
- Aufarbeitung durch Fällen
   Das klare Filtrat der vom Katalysator befreiten Lösung oder eine Lösung dews durch Ionenaustauscher von Polyol gereinigten Gemisches entsprechend Formel II oderIIIwird bis zur öligen Konsistenz am Rotationsverdampfer im Vakuum bei ca. 40 - 50°C eingeengt und dem Öl noch in der Wärme Methanol zugesetzt. Im Falle der Darstellung der Verbindungen entsprechend Formel II oder III fällt beim Abkühlen eine Mischung der Verbindungen aus, in der das jeweilige Mannitisomere überwiegt. Durch sukzessive Zugabe von Aceton wird eine aus überwiegend den Sorbitisomeren bestehende zweite Charge erhalten. Die Fällung wird mit Ether vervollständigt. Im Falle der Darstellung der Verbindung gemäß Formel I wird nach der Methanolzugabe zunächst der Lösung Aceton bis zur Trübung und dann bei ca. 4°C eine Mischung aus Aceton/Ether bis zur möglichst vollständigen Fällung des Reaktionsproduktes zugesetzt.
   Zu beachten ist, daß im Falle der Darstellung von Verbindungen gemäß FormelIII im Reaktionsgemisch nach der reduktiven Aminierung bereits das Mannitisomere überwiegen kann.

### Beispiel 2

Katalytische reduktive Aminierung von Isomaltulose mit Ammoniak in Methanol.

In einem Druckgefäß wird eine Suspension, bestehend aus bei 0° mit Ammoniak gesättigtem Methanol (300 ml), 20 g Isomaltulose, ca. 50 ml Molekularsiebe (3A°) und 0,56 g NH₄Cl 6 Tage bei Raumtemperatur gerührt. In dieser Zeit löst sich die Isomaltulose vollständig. Nach der Reaktion werden die Molekularsiebe abfiltriert und die Lösung in einem Autoklaven mit methanolischem Raney-Nickel bei 50°C und ca. 120 bar Wasserstoffdruck 12 Std. hydriert. Nach dem Abfiltrieren des Katalysators fällt langsam eine erste Produktfraktion aus, die vorwiegend das Mannitisomere enthält. Die weitere Reinigung und Aufarbeitung erfolgt wie in Beispiel 1 beschrieben. Ausbeute 7,2 g = 36% d. Theorie.

### Beispiel 3

Reduktive Aminierung von Isomaltulose mit einem komplexen Metallhydrid.

40 ml einer wie in Beispiel 2 nach 6 Tagen Rühren erhaltenen Lösung wird zur Trockne abdestilliert und der Rückstand zu einer wäßrigen Lösung von Natriumborhydrid (0,4 g) gegeben. Nach ca. 16-stündigem Rühren bei Raumtemperatur wird die Lösung mit Salzsäure auf pH 6 eingestellt und zur Trockne abdestilliert. Der Rückstand wird mehrfach nach Zugabe von Methanol zur Trockne eingeengt bis alles Borat entfernt ist. Die Reinigung des boratfreien Rückstandes erfolgt mit Kationenaustauscher, wie in Beispiel 1 beschrieben. (Ausbeute 1,3 g = 52% der Theorie).

### Beispiel 4

Darstellung von 2-Amino-(1ʹ→6)-GPS aus Isomaltulose durch Heyns-Umlagerung.

In Anlehnung an die Darstellung von Glucosamin aus Fructose (K. Heyns; H. Paulsen; R. Eichstädt und M. Rolle in Chem.Berichte 90 (1957) S. 2039) wird aus Isomaltulose über Isomaltosamin - das als Zwischenprodukt nicht isoliert, sondern unmittelbar hydriert wird - 2-Amino-(1ʹ→6)-GPS dargestellt.

Eine Suspension von 5 g Isomaltulose wird, wie in Beispiel 2 beschrieben, in Gegenwart von Molekularsieben und Ammoniumchlorid in ammoniakalischem Methanol 6 Tage bei Raumtemperatur gerührt. Die erhaltene Lösung wird nach Abfiltrieren der Molekularsiebe bis zur Trockne eingeengt und der Rückstand zusammen mit 3,5 g Bernsteinsäure in 100 ml abs. Methanol erneut aufgenommen.
Die Suspension wird 4 Tage bei +4°C gerührt, danach filtriert und das klare Filtrat zur Trockne eingeengt. Dieses Rohprodukt wird in dest. Wasser gelöst, über Nacht bei Raumtemperatur gerührt, und die Lösung dann auf eine Kationenaustauschersäule (z. B. IR 120, Fa. Amberlite, (H⁺-Form) 200 ml) gegeben.

Die Säule wird mit ca. 1 l Wasser zuckerfrei gewaschen und anschließend mit 1 l 0,5 n Trifluoressigsäure eluiert. Das Eluat wird mit Ether durch Exktraktion weitgehend von Trifluoressigsäure befreit und zur Trockne eingeengt (1,6 g Rohprodukt).
Das Rohprodukt wird in wenig Wasser gelöst, mit wäßrigem Ammoniak auf ca. pH 8 eingestellt und dieser Lösung 0,25 g Natriumborhydrid zugesetzt. Das Reaktionsprodukt, wird wie in Beispiel 3 beschrieben, aufgearbeitet. Ausbeute 300 mg (ca. 6%).

Eine chromatographische Untersuchung bestätigte, daß 2-Amino-(1ʹ→6)-GPS entstanden war (analytische Bedingungen siehe Beispiel 6).

### Beispiel 5

Darstellung eines Aminopolyolgemisches aus Palatinosemelasse.

Bei der Gewinnung von Palatinose durch Kristallisation fällt als Endablauf Palatinosemelasse an. Diese Melasse wird, wie in der DE-OS 32 41 788 beschrieben, durch Fermentation von Glucose, Fructose und Saccharose befreit. Die Hefe wird separiert. Eine so erhaltene aufbereitete Lösung wird nach Entfärbung mittels Entfärberharz und/oder Aktivkohle entsprechend Beispiel 1 reduktiv aminiert.
Nach erneuter Entfärbung mittels Aktivkohle kann die so erhaltene Lösung, wie unter Beispiel 1 beschrieben, über Ionenaustauscher oder durch Fällung mit Methanol aufgearbeitet werden. Die entstandene Aminopolyolmischung enthält, wie eine chromatographische Untersuchung (s. Beispiel 6) zeigt, die Aminopolyole entsprechend Formeln I - III.

### Beispiel 6

Die Identität der durch die verschiedenen Verfahren erhaltenen Aminopolyole gemäß Formlen I - III wurde durch Hochdruckflüssigkeitschromatographie (Ionenpaarchromatographie) nachgewiesen.

Chromatographiebedingungen:
- Säule:: RP 18 Kartusche, Fa. Merck, 250-4; 7 µm
- mob. Phase:: ca. 2 g Ammoniumheptansulfonat/l Wasser
- Temperatur:: 20°C
- Fluß:: 0,7 cm³/min
- Detektor:: Differentialrefraktometer

Nach ca. 21 min Chromatographiezeit tritt im Chromatogramm ein Peak auf, der freigesetztem Ammonium entspricht. Die Aminopolyole folgen zeitlich hinter diesem Peak in der Reihenfolge:
- 2-Amino-1ʹ→1-GPS
- 2-Amino-1ʹ→1-GPM
- 2-Amino-1ʹ→6-GPS
- 1-Amino-1ʹ→6-GPS
- 2-Amino-1ʹ→6-GPM

Die Chromatographiebedingungen lassen sich auf eine präparative Hochdruckchromatographiesäule zur Trennung der isomeren Gemische übertragen. Zur Aufarbeitung der reinen Verbindungen gemäß Formeln II und III wird dann das Ionenpaarreagenz mit einem Anionenaustauscher (z. B. IRA 400, Fa. Amberlite, (OH-Form))entfernt. Nach Einengen bis zur Trockene erhält man die reinen Verbindungen.

Die nachstehenden Beispiele 7 - 9 beschreiben die Darstellung der N-Acylaminopolyole.

### Beispiel 7

Selektive N-Acylierung in wäßriger Lösung mit einem kurzkettigen Säurechlorid

53 g Aminopolyol entsprechend Formel I,II oder III (0,16 Mol) werden mit 21,3 g (0,16 Mol) Kaliumkarbonat und 0,7 g Natriumnitrit in Wasser gelöst. Unter starkem Rühren und Kühlen (Tₘₐₓ = 5°C) wird Acrylsäurechlorid zugesetzt, anschließend 1 Stunde bei 5°C und danach weitere 10 - 15 Stunden bei Raumtemperatur weiter gerührt.

Nach Fällung mit ca. 1 l Ethanol werden die anorganischen Salze abfiltriert. Dem klaren Filtrat wird zunächst bei Raumtemperatur bis zur Trübung, später bei +4°C sukzessive eine Mischung aus Aceton/Ether zugesetzt, bis das Reaktionsprodukt möglichst vollständig gefällt ist.

Isomerenfreie Produkte erhält man durch Kristallisation. Setzt man z. B. eine überwiegend aus 2-Amino-(1ʹ→6)-GPS bestehende Mischung (Herstellung s. Beispiel 1) ein, erhält man nach Lösen des gefällten Produktes in wenig 50%igem Methanol und sukzessiver Zugabe von Ethanol, Aceton und Ether das Sorbitisomer in nadelförmigen Kristallen (Ausbeute ca. 60%).

### Beispiel 8

Selektive N-Acylierung mit einem kurzkettigen verzweigten Säureanhydrid in Methanol
66,6 g (0,2 mol) Aminopolyol entsprechend Formel I, II oder III dargestellt nach Beispiel 1 werden in 200 ml abs. Methanol suspendiert bzw. gelöst. Die Lösung wird auf - 5 °C abgekühlt und unter Konstanthalten der Temperatur langsam mit 32 g (0,2 mol) Methacrylsäureanhydrid versetzt. Danach wird für 18 h bei 0°C weitergerührt.

Durch Zugabe von Aceton/Ether wird das Reaktionsprodukt gefällt. Das pulverförmig anfallende Amid wird in wenig Wasser aufgenommen und mit Ethanol/Aceton/Ether analog Beispiel 7 kristallisiert.
(Ausbeute ca. 70 %).

### Beispiel 9

Selektive N-Acylierung mit einem langkettigen Säureanhydrid in Methanol in Gegenwart eines basischen Ionenaustauschers.

3 g Aminopolyol entsprechend Formel I,II oder III, dargestellt nach Beispiel 1, werden zusammen mit 25 ml mit Wasser und Methanol gewaschenem basischen Ionenaustauscher (z. B. IRA 400, Fa. Amberlite (OH-Form) und 4,3 g Palmitinsäureanhydrid in 100 ml abs. Methanol suspendiert und 48 Stunden bei Raumtemperatur gerührt. Nach Abdestillieren des Methanol wird der Rückstand in einer Soxhlett-Apparatur mit Petrolether extrahiert. Der so erhaltene Rückstand wird mit heißem Butanol extrahiert und filtriert. Das Filtrat wird dreimal mit einer 5%igen wäßrigen Kochsalzlösung ausgeschüttelt und mit Magnesiumsulfat getrocknet. Nach dem Abdestillieren ist der Rückstand im allgemeinen dünnschichtchromatographisch reines Aminopolyol-N-Acylat. Eventuell sich im IR-Spektrum noch zeigende Spuren freier Fettsäure können durch kurzes erneutes Extrahieren mit Petrolether entfernt werden.
Ausbeute ca. 50 - 60% der Theorie.

### Beispiel 10

Biologischer Abbauversuch der erfindungsgemäßen Verbindungen

Die biologische Abbaubarkeit wird nach DEV H5/Teil 2 (Deutsches Einheitsverfahren für Wassser-, Abwasser- und Schlammuntersuchung) untersucht.
Eingesetzt werden
- 1ʹ→6-Isomaltamin-(2)
- 1ʹ→6-Isomaltamin-(2)-N-palmitat
- 1ʹ→6-Isomaltamin-(2)-N-acrylat-polymer.

Der biologische Sauerstoffbedarf wurde bei jeweils 2 Verdünnungen gemessen. Ein Sauerstoffverbrauch wurde nicht festgestellt.

### Beispiel 11

Darstellung eines Aminopolyol-N-acryl-polymers.

3 g 1ʹ→6-Isomaltamin-(2)-N-acrylamid werden in 10 ml entgastem bidest. Wasser gelöst und 1 Tropfen Eisessig zugesetzt. Die Lösung wird in einem mit nachgereinigtem Stickstoff begasten thermostatisierbaren Reaktionsgefäß zubereitet. 46 mg (NH₄)₂S₂O₈ als oxidative und 11 mg Na₂S₂O₅ als reduktive Initiatorkomponente werden separat in jeweils einigen Tropfen entgastem bidest. Wasser gelöst und nacheinander in das Reaktionsgefäß durch ein Septum eingespritzt.
Die Polymerisation erfolgt bei +4°C innerhalb 3 - 6 Stunden.
Bei Ausbeuten von 30 - 60% werden Molekulargewichte im Bereich von 10⁵ bis 10⁶ erreicht.

## Patentansprüche

1. Isomaltamine sowie deren N-Acylderivate entsprechend den Formeln 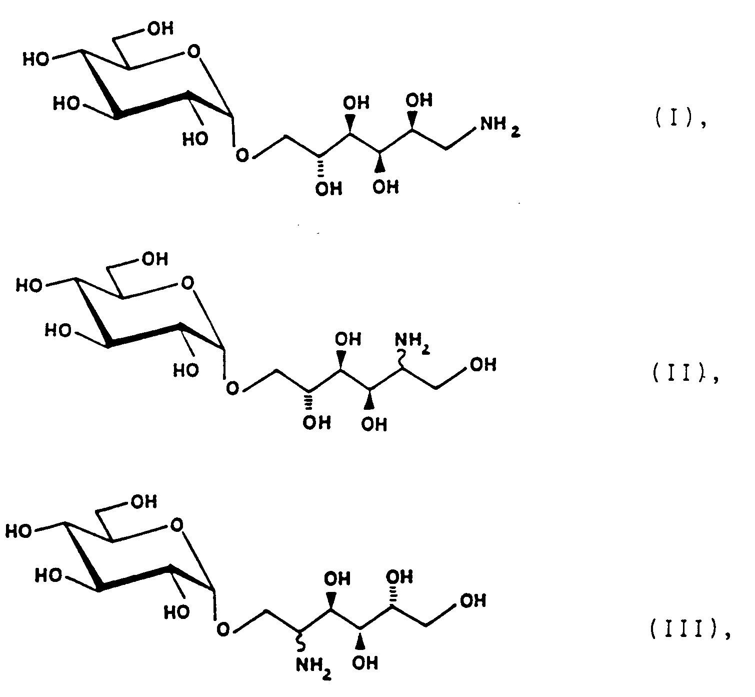 Mischungen der Isomeren in beliebigen Verhältnissen, sowie 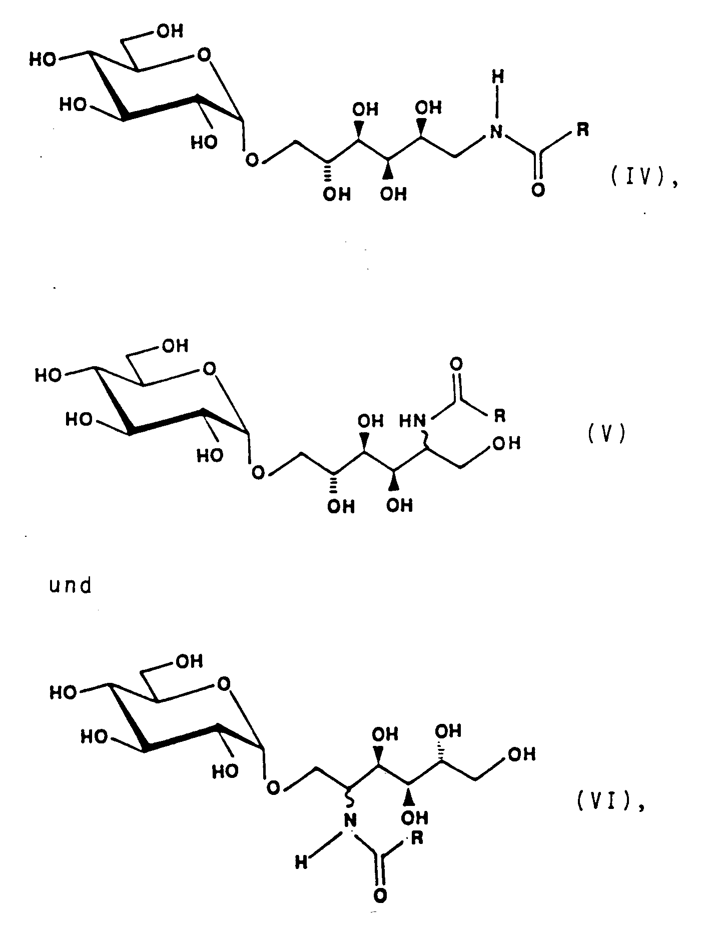 wobei R ein verzweigter oder unverzweigter, gesättigter oder ungesättigter Alkylrest mit 2 - 21 C-Atomen oder eine Vinyl-, Propenyl-2-, Propenyl-2-carbonsäure- oder Vinylcarbonsäuregruppe bedeutet.

2. Verfahren Zur Herstellung der Isomaltamine gemäß Anspruch 1, gekennzeichnet durch reduktive Aminierung von Isomaltose, Isomaltulose bzw. α-D-Glucopyranosyl-(1→1)-D-fructose mittels Ammoniak oder Hydrazinderivaten, vorzugsweise Hydrazin, entweder in Gegenwart von Katalysatoren, vorzugsweise Raney-Nickel, und Wasserstoff oder mittels komplexen Metallhydriden, z.B. Natriumborhydrid, in wäßriger, wäßrig-alkoholischer oder alkoholischer Lösung bzw. Suspension, der sich die Isolierung z.B. durch fraktioniertes Fällen mittels geeigneten Lösungsmitteln, wie Methanol, Aceton und Ether, und/oder Reinigung mittels saurer Kationenaustauscher anschließt.

3. Verfahren zur Herstellung der N-Acylderivate der Isomaltamine, gekennzeichnet durch selektive N-Acylierung der Verbindungen der Formeln I, II oder III bzw. Mischungen dieser Verbindungen in beliebigen Verhältnissen in an sich bekannter Weise mittels Säurehalogeniden oder Säureanhydriden in wäßriger, wäßrig-alkoholischer oder alkoholischer Lösung bzw. Suspension, gegebenenfalls in Gegenwart einer basischen Substanz, wie Kaliumcarbonat, Natriumcarbonat oder eines basischen Ionenaustauschers, mit anschließender Isolierung und Reinigung der so erhaltenen Verbindungen entweder durch Fällung, Kristallisation oder durch Extraktion.

4. Verwendung der N-Acylderivate nach Anspruch 1 mit einem Alkylrest zwischen 5 und 21 C-Atomen entsprechend ihrem HLB-Wert (hydrophilic lipophillc balance) als hydrophiles Tensid, z.B. als Netzmittel, Emulgator oder waschaktive Substanz.

5. Verwendung der N-Acylderivate nach Anspruch 1 mit Acylresten von Acryl-, Methacryl-, Itacon- oder Maleinsäure zur Herstellung hydrophiler Polymerer durch Homo- oder Copolymerisation.

## Claims

1. Isomaltamines and their N-acyl derivatives corresponding to the formulae 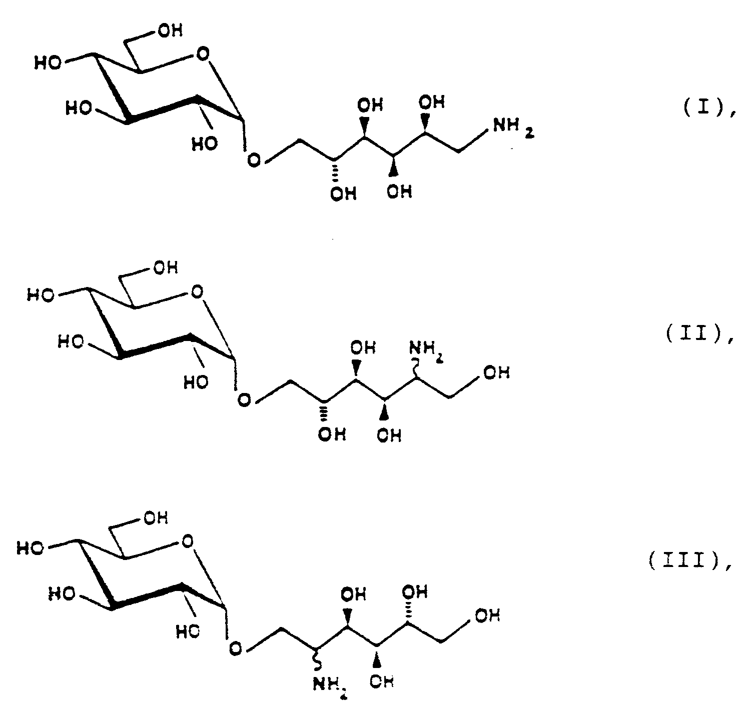 mixtures of the isomers in any proportions, as well as 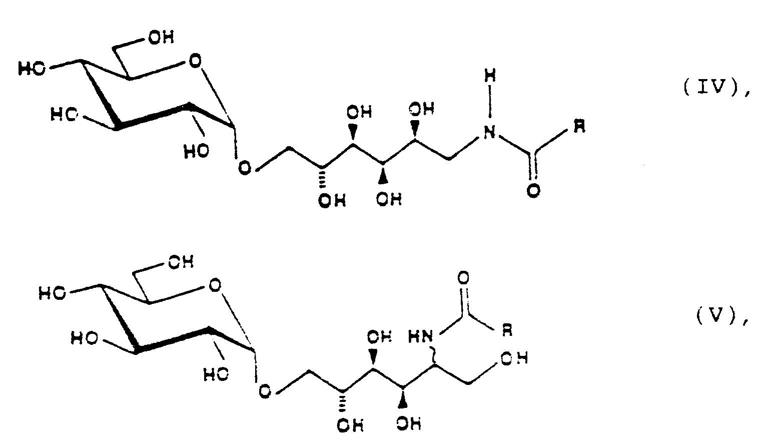 and 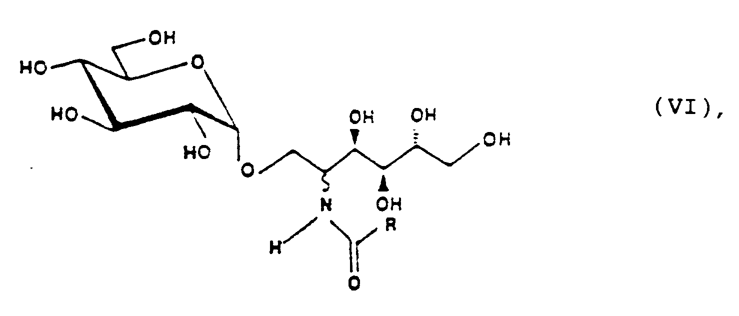 whereby R signifies a branched or non-branched, saturated or unsaturated alkyl residue with 2 - 21 C-atoms or a vinyl group, propenyl-2 group, propenyl-2-carboxylic acid group or vinyl carboxylic acid group.

2. Method for producing the isomaltamines according to claim 1, characterized by reductive amination of isomaltose, isomaltulose or α-D-glucopyranosyl-(1→1)-D-fructose by means of ammonia or hydrazine derivatives, preferably hydrazine, either in the presence of catalysts, preferably Raney nickel, and hydrogen or by means of complex metal hydrides, for example sodium borohydride, in aqueous, aqueous-alcoholic or alcoholic solution or suspension, which is followed by the isolation, for example through fractionated precipitation by means of suitable solvents, such as methanol, acetone and ether, and/or purification by means of acidic cationic exchanger.

3. Method for producing the N-acyl derivatives of the isomaltamines, characterized by selective N-acylation of the compounds of the formulae I, II or III or mixtures of these compounds in any proportions in a manner known in itself by means of acid halides or acid anhydrides in aqueous, aqueous-alcoholic or alcoholic solution or suspension, possibly in the presence of a basic substance, such as potassium carbonate, sodium carbonate or a basic ion exchanger, with subsequent isolation and purification of the compounds obtained in this way either by precipitation, crystallization or by extraction.

4. Use of the N-acyl derivatives according to claim 1 with an alkyl residue between 5 and 21 C-atoms corresponding to their HLB value (hydrophilic lipophilic balance) as hydrophilic tenside, for example as wetting agent, emulsifier or surface-active substance.

5. Use of the N-acyl derivatives according to claim 1 with acyl residues of acrylic acid, methacrylic acid, itaconic acid or maleic acid for producing hydrophilic polymers by means of homopolymerization or copolymerization.

## Revendications

1. Isomaltamines ainsi que leurs dérivés N-acyl correspondant aux formules 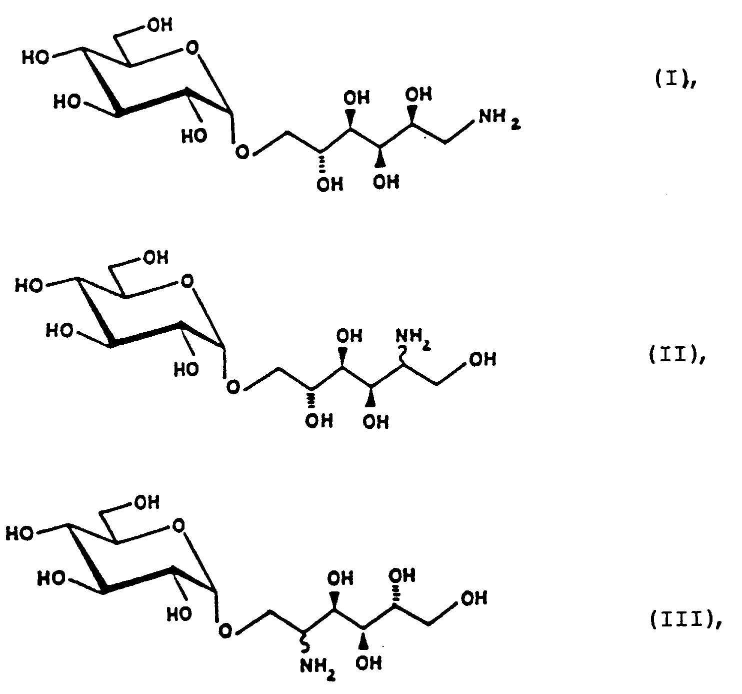 Mélanges des isomères en proportions quelconques, ainsi que 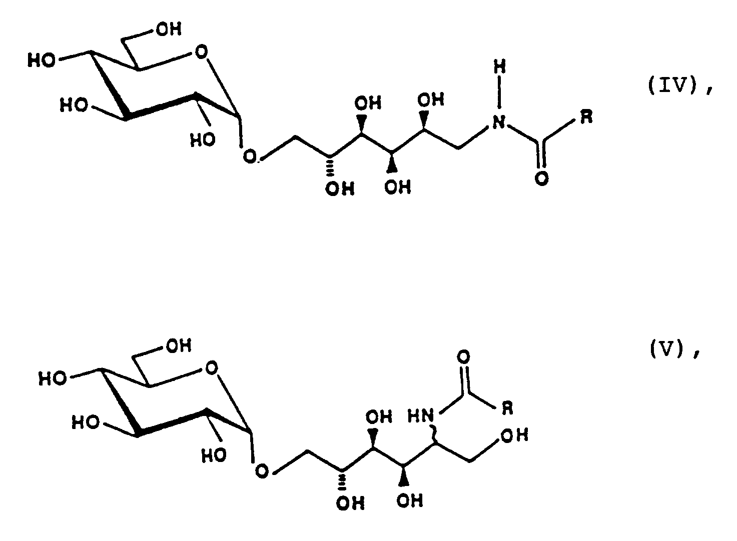 et 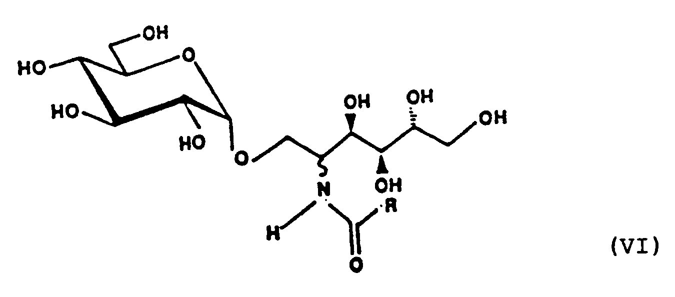 dans lesquelles R représente un reste alkyl ramifié ou non-ramifié, saturé ou insaturé avec 2 à 21 atomes C ou un groupe vinyl-, propényl-2, acide carbonique de propényl-2 ou acide carbonique de vinyle.

2. Procédé de préparation des isomaltamines selon la revendication 1, caractérisé par une aminisation réductive d'isomaltulose ou de α-D-glucopyranosyl-(1→1)D-fructose au moyen d'ammoniac ou de dérivés de l'hydrazine, soit en présence de catalyseurs, de préférence du nickel de Raney, et d'hydrogène, ou au moyen d'hydrures métalliques complexes, par exemple boro-hydrure de sodium, en solution ou en suspension aqueuse, aqueuse-alcoolique ou alcoolique, à laquelle aminisation fait suite l'isolation, par exemple par précipitation fractionnée au moyen de solvants appropriés tels que méthanol, acétone et éther, et/ou une purification au moyen d'un échangeur de cations acide.

3. Procédé de préparation des dérivés N-acyl des isomaltamines, caractérisé par une N-acylisation sélective des composés des formules I, II ou III ou de mélanges de ces composés en proportions quelconques, d'une manière connue en soi, au moyen d'halogénures acides ou d'anhydrides acides en solution ou suspension aqueuse, aqueuse-alcoolique ou alcoolique, le cas échéant en présence d'une substance basique telle que le carbonate de potassium, le carbonate de sodium ou d'un échangeur d'ions basique, à laquelle font suite l'isolation et la purification des composés ainsi obtenus, soit par précipitation, cristallisation, soit par extraction.

4. Utilisation des dérivés N-acyl selon la revendication 1, avec un reste alkyl entre 5 et 21 atomes C selon leur valeur HLB (hydrophilic lipophilic balance) en tant qu'agents tensio-actifs hydrophiles, par exemple en tant qu'agents de réticulation, d'émulsifiants ou de substances actives pour le lavage.

5. Utilisation des dérivés N-acyl selon la revendication 1 avec des restes acyl d'acide acrylique, méthacrylique, itaconique ou maléinique pour la préparation de polymères hydrophiles, par homopolymérisation ou par copolymérisation.
